# EUROPEAN PATENT SPECIFICATION

(11) **EP 3 730 137 B1**
(45) Date of publication and mention of the grant of the patent: **20.03.2024**
(21) Application number: 18891086.3
(22) Date of filing: 20.12.2018
(51) Int. Cl.: A61K 31/335, A61K 31/5377, A61K 9/06, A61K 9/08, A61K 31/138, A61K 31/343, A61K 31/353, A61K 31/4704, A61P 27/02, A61P 27/06, A61K 9/00, A61K 47/02, A61K 47/12, A61K 47/26

(54) **THERAPEUTIC AGENT FOR GLAUCOMA COMPRISING AN FP AGONIST AND TIMOLOL**
THERAPEUTIKUM FÜR GLAUKOM MIT EINEM FP-AGONISTEN UND TIMOLOL
AGENT THÉRAPEUTIQUE DU GLAUCOME COMPRENANT UN AGONISTE FP ET TIMOLOL

(30) Priority: 21.12.2017 JP 2017244573
(43) Date of publication of application: 28.10.2020
(73) Proprietor: ONO PHARMACEUTICAL CO., LTD., Osaka-shi, Osaka 541-8526 (JP)
(72) Inventor: YAMANE, Shinsaku, Mishima-gun, Osaka 618-8585 (JP); NAKAYAMA, Satoshi, Mishima-gun, Osaka 618-8585 (JP); NAGAI, Kazufumi, Mishima-gun, Osaka 618-8585 (JP); MORIYUKI, Kazumi, Mishima-gun, Osaka 618-8585 (JP); KARAKAWA, Tomohiro, Mishima-gun, Osaka 618-8585 (JP)
(74) Representative: Grünecker Patent- und Rechtsanwälte PartG mbB
(86) International application number: PCT/JP2018/047102
(87) International publication number: WO 2019/124523

(56) References cited:
- WO-A1-2011/013651
- US-A1- 2016 287 551
- LOUISE J LU ET AL: "Novel Pharmacologic Candidates for Treatment of Primary Open-Angle Glaucoma", THE YALE JOURNAL OF BIOLOGY AND MEDICINE, vol. 90, no. 1, March 2017 (2017-03), pages 111-118, XP055765723, US ISSN: 0044-0086
- LUCIANO QUARANTA ET AL: "Prostaglandin Analogues (PGAs) And Timolol Fixed Combination (Fc) Vs. Extemporaneous Combination (Ec) Or Monotherapy (Mt) In The Treatment Of Primary Open-angle Glaucoma (POAG) And Ocular Hypertension (OHT): A Systemtic Review And Meta- analysis", INVESTIGATIVE OPHTHALMOLOGY & VISUAL SCIENCE, vol. 52, no. abstract 215, April 2011 (2011-04), XP055765804,

## Description

### TECHNICAL FIELD

The present invention, in one embodiment, relates to an agent for use in a method of treating glaucoma or ocular hypertension, the method comprising administering the agent comprising an FP (prostaglandin F) agonist as an active ingredient administered in combination with timolol or a pharmaceutically acceptable salt thereof, wherein
the FP agonist is 2-propanyl 4-{(3S,5aR,6R,7R,8aS)-6-[(1E,3R)-4-(2,5-difluorophenoxy)-3-hydroxy-1 -buten-1-yl]-7-hydroxyoctahydro-2H-cyclopenta[b]oxepin-3-yl}butanoate, and wherein timolol or a pharmaceutically acceptable salt thereof and 2-propanyl 4-{(3S,5aR,6R,7R,8aS)-6-[(1E,3R)-4-(2,5-difluorophenoxy)-3-hydroxy-1-buten-1-yl]-7-hydroxyoctahydro-2H-cyclopenta[b]oxepin-3-yl}butanoate are administered separately or concurrently.

The FP agonist is a compound represented by the following formula: 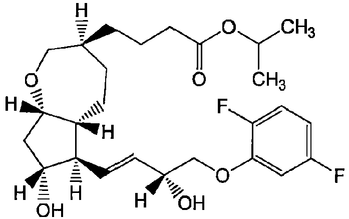 wherein all symbols have the same meanings as those described later.

### BACKGROUND ART

Glaucoma is an eye disease characterized by disturbance of visual function causing transient or permanent visual field defect and reduced visual acuity. Aqueous humor is accumulated due to disturbance of aqueous humor circulation, and intraocular pressure is continuously increased, resulting in compression of the optic nerve. In the treatment of glaucoma, it is effective to lower intraocular pressure, and for example, drug therapy (eye drops, internal medicine, and infusion treatment), laser therapy, and surgical therapy are performed to lower intraocular pressure.

An FP agonist which is used as an active ingredient in the present invention is known as a medicine useful as an agent for preventing and/or treating eye diseases and the like (see Patent Literature 1). Among others, 2-propanyl 4-{(3S,5aR,6R,7R,8aS)-6-[(1E,3R)-4-(2,5-difluorophenoxy)-3-hydroxy-1-buten-1-yl]-7-hydroxyoctahydro-2H-cyclopenta[b]oxepin-3-yl}butanoate is known as Sepetaprost (International Nonproprietary Name) (hereinafter sometimes abbreviated as Compound A) and the development as a therapeutic agent for glaucoma is underway.

At the treatment site of glaucoma, it is common to use drugs with different mechanisms of intraocular pressure lowering in combination, and for example, Xalacom (registered trademark) Combination Eye Drops (latanoprost-timolol maleate combination), Tapcom (registered trademark) Combination Eye Drops (tafluprost-timolol maleate combination) and the like are commercially available.

On the other hand, Patent Literature 1 describes Compound A as Example 16 (25), and as an example of other drugs for complementing and/or enhancing preventing and/or treating effects on glaucoma of Compound A and the like, β-blockers are described. Louise J Lu et al., Novel Pharmacologic Candidates for Treatment of Primary Angle Glaucoma, The Yale Journal of Biology and Medicine, vol. 90, no.1, March 2017, pages 111-118, discloses the use of ONO-9054 in the treatment of glaucoma and intraocular pressure (IOP).

### CITATIONS LIST

Patent Literature 1: WO 2011/013651

### SUMMARY OF INVENTION

### TECHNICAL PROBLEMS

An object of the present invention is to find effective therapy for glaucoma and to provide as a medicine.

### SOLUTIONS TO PROBLEMS

The present inventors have conducted intensive studies to achieve the object, and as a result, have found that the object can be achieved by a combination of the FP agonist 2-propanyl 4-{(3S,5aR,6R,7R,8aS)-6-[(1E,3R)-4-(2,5-difluorophenoxy)-3-hydroxy-1-buten-1-yl]-7-hydroxyoctahydro-2H-cyclopenta[b]oxepin-3-yl}butanoate and the β-blocker timolol (hereinafter, sometimes abbreviated as the combination of the present invention).

The present invention provides the following embodiments.

In a first embodiment, an agent for use in a method of treating glaucoma or ocular hypertension is provided , the method comprising administering the agent comprising 2-propanyl 4-{(3S,5aR,6R,7R,8aS)-6-[(1E,3R)-4-(2,5-difluorophenoxy)-3-hydroxy-1-buten-1-yl]-7-hydroxyoctahydro-2H-cyclopenta[b]oxepin-3-yl}butanoate as an active ingredient administered in combination with timolol or a pharmaceutically acceptable salt thereof, wherein timolol or a pharmaceutically acceptable salt thereof and 2-propanyl 4-{(3S,SaR,6R,7R,8aS)-6-[(1E,3R)-4-(2,5-difluorophenoxy)-3-hydroxy-1-buten-1-yl]-7-hydroxyoctahydro-2H-cyclopenta[b]oxepin-3-yl}butanoate are administered separately or concurrently.

In a second embodiment, a pharmaceutical composition for use in a method of treating glaucoma or ocular hypertension is provided, the method comprising administering the pharmaceutical composition comprising 2-propanyl 4-{(3S,5aR,6R,7R,8aS)-6-[(1E,3R)-4-(2, 5-difluorophenoxy)-3-hydroxy-1-buten-1-yl]-7-hydroxyoctahydro-2H-cyclopenta[b]oxepin-3-yl}butanoate in combination with timolol or a pharmaceutically acceptable salt thereof.

The pharmaceutical composition for use is preferably an eye drop or an eye ointment.

The pharmaceutical composition for use more is preferably a compounding agent.

### EFFECTS OF INVENTION

The combination of the present invention is useful as an agent for treating glaucoma since the combination enhances intraocular pressure lowering action compared to a single administration of each drug and has an effect of maintaining intraocular pressure lowering action.

### DESCRIPTION OF EMBODIMENTS

### (1) FP agonist

The FP agonist used in the combination of the present invention, is 2-propanyl 4-{(3S,5aR,6R,7R,8aS)-6-[(1E,3R)-4-(2,5-difluorophenoxy)-3-hydroxy-1-buten-1-yl]-7-hydroxyoctahydro-2H-cyclopenta[b]oxepin-3-yl}butanoate represented by the following formula described in WO 2011/013651:

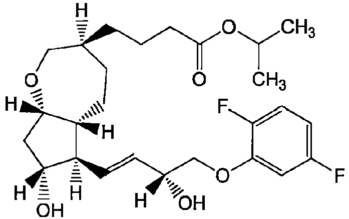

This compound is also described as compound A, herein.

Further described herein is a compound represented by the general formula (I) described in WO 2011/013651: 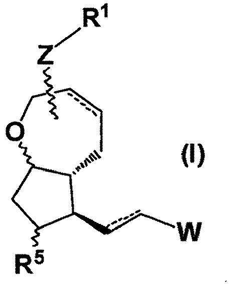 wherein all symbols have the same meaning as those described in WO 2011/013651, a salt thereof, a solvate thereof, or a prodrug thereof is mentioned.

In the present invention, all isomers are included unless otherwise indicated. For example, alkyl groups include straight-chain and branched-chain ones. Furthermore, geometric isomers at double bond, ring, fused ring (E-form, Z-form, cis-form, trans-form), optical isomers due to the presence of asymmetric carbon atom and the like (R and S configurations, α and β configurations, enantiomers, diastereomers), optically active substances having optical activity (D-form, L-form, d-form, l-form), polar substances derived from chromatographic separation (highly polar substances, lowly polar substances), equilibrated compounds, rotamers, mixtures of them in any proportion, and racemic mixtures are all included in the present invention. In addition, all isomers by tautomerism are also included in the present invention.

Further, optical isomers in the present invention may include not only 100% pure ones but also other optical isomers of less than 50% pure.

2-Propanyl 4-{(3S,5aR,6R,7R,8aS)-6-[(1E,3R)-4-(2,5-difluorophenoxy)-3-hydroxy-1-buten-1-yl]-7-hydroxyoctahydro-2H-cyclopenta[b]oxepin-3-yl}butanoate (Compound A) can be prepared according to Example 1 → Example 2 → Example 3 → Example 4 → Example 5 —> Example 6 —> Example 7 —> Example 8 —> Example 9 —> Example 10 (1) —> Example 11 —> Example 12 —> Example 13 —> Example 14 —> Example 15 —> Example 16 (25) described in WO 2011/013651.

A dose of Compound A used in the combination of the present invention varies depending on age, body weight, symptom, therapeutic effect, administration method, treatment time, and the like. For example, in the case of eye drops, one to a few drops per dropping having a concentration of preferably 0.000001 to 5% (w/v), more preferably 0.00001 to 0.05% (w/v) as a one time amount may be dropped into eyes once to a few times (for example, 1 to 8 times) per day per adult. In addition, in the case of an eye ointment, the eye ointment having a concentration of preferably 0.000001 to 5% (w/w), more preferably 0.00001 to 0.05% (w/w) may be applied once to a few times (for example, 1 to 4 times) per day.

### (2) β-blocker

In the present disclosure, a β-blocker is not particularly limited as long as it is a drug that blocks any one of β-adrenergic receptors, that is, each of β1, β2 and β3-receptors among adrenergic receptors of sympathetic nerves, and is used for treating glaucoma. For example, timolol, carteolol, levobunolol, betaxolol, nipradilol, befunolol, or pharmaceutically acceptable salts thereof can be mentioned. In the combination of the invention, timolol is used, and timolol maleate is preferred.

The dose of the β-blocker used in the combination of the present invention varies depending on age, body weight, symptom, therapeutic effect, administration method, treatment time and the like, but a dose obtained a manufacturing approval as a medicine or a lower dose than the dose can be applied. Specific doses for timolol include 0.1 to 0.5% (w/v), preferably 0.25 to 0.5% (w/v). Specific doses for carteolol include 0.1 to 2% (w/v), preferably 1 to 2% (w/v). Specific doses for levobunolol include 0.1 to 0.5% (w/v), preferably 0.25 to 0.5% (w/v). Specific doses for betaxolol include 0.1 to 0.5% (w/v), preferably 0.25 to 0.5% (w/v). Specific doses for nipradilol include 0.1 to 0.25% (w/v), preferably 0.25% (w/v). Specific doses for befunolol include 0.1 to 0.25% (w/v), preferably 0.25% (w/v).

In the present disclosure, the salt is preferably a pharmaceutically acceptable salt, and is preferably a water-soluble salt. Examples of the pharmaceutically acceptable salt include a salt of an alkali metal (potassium, sodium and the like), a salt of an alkaline earth metal (calcium, magnesium and the like), an ammonium salt, a salt of a pharmaceutically acceptable organic amine (tetramethylammonium, triethylamine, methylamine, dimethylamine, cyclopentylamine, benzylamine, phenethylamine, piperidine, monoethanolamine, diethanolamine, tris(hydroxymethyl)aminomethane, lysine, arginine, N-methyl-D-glucamine and the like), an acid adduct salt (an inorganic acid salt (hydrochloride, hydrobromide, hydroiodide, sulfate, phosphate, nitrate and the like), an organic acid salt (acetate, trifluoroacetate, lactate, tartrate, oxalate, fumarate, maleate, benzoate, citrate, methanesulfonate, ethanesulfonate, benzenesulfonate, toluenesulfonate, isethionate, glucuronate, gluconate and the like) and the like) and the like.

Since all of the above-described β-blockers are commercially available compounds, their toxicity is sufficiently low and they can be used safely as medicines.

Since the combination of the present invention is used for the treatment of glaucoma or ocular hypertension, both the FP agonist and the β-blocker are preferably in dosage forms suitable for the treatment of the diseases, for example, an eye drop, an eye ointment and the like. Each of them may be in a separate dosage form. In addition, the combination of the present invention may be separate preparations, and a compounding agent containing both agents in appropriate amounts, for example, a combination eye drop and a combination eye ointment are also preferable in consideration of medication compliance.

In the combination of the present invention, when the FP agonist and the β-blocker are used in separate preparations, simultaneous administration and administration with a time difference are included. In addition, administration with a time difference means that the FP agonist and the β-blocker are separately administered at a certain time interval. As the order of administration, the FP agonist may be administered first and the β-blocker may be administered later, or the β-blocker may be administered first and the FP agonist may be administered later.

The eye drop and the eye ointment used in the combinations of the present invention can be formulated using commonly used techniques. For example, in the case of eye drops, an isotonizing agent, a buffer agent, a pH-adjusting agent, a solubilizing agent, a thickener, a stabilizer, a preservative, and the like can be appropriately added as additives. In addition, a stable eye drop can be obtained by adding a pH-adjusting agent, a thickener, a dispersing agent and the like and suspending the drug.

### [Toxicity]

The toxicity of the combination of the present invention is sufficiently low and the combination of the present invention can be safely used as a medicine.

### [Application to medicine]

One embodiment of diseases treated by the combination of the present invention includes glaucoma and ocular hypertension. Examples of glaucoma include acute angle-closure glaucoma, chronic angle-closure glaucoma, secondary angle-closure glaucoma, primary open-angle glaucoma, secondary open-angle glaucoma, congenital glaucoma, normal tension glaucoma, and hypersecretion glaucoma.

In the present invention, the combination of the present invention may be administered in combination with another agent for treating glaucoma for: (1) complementation and/or enhancement of therapeutic effects; (2) improvement of kinetics and absorption, lowering of a dose; and/or (3) alleviation of side effects.

In the present invention, the other agent for treating glaucoma may be any known agent. Examples of the other agent for treating glaucoma include a sympathetic agent (an α2 agonist: for example, apraclonidine hydrochloride and the like, a β2 agonist: for example, dipivefrine hydrochloride and the like), a parasympathomimetic agent (for example, pilocarpine hydrochloride, carbachol, demecarium, echothiophate or distigmine bromide and the like), a sympathetic inhibitor (an α1-blocker: for example, bunazosin hydrochloride and the like), a prostaglandin drug (for example, isopropyl unoprostone, latanoprost, bimatoprost, travoprost, an EP2 agonist, an EP4 agonist or a DP agonist and the like), a carbonic anhydrase inhibitor (for example, acetazolamide, diclofenamide, methazolamide, dorzolamide hydrochloride, or brinzolamide and the like), a hyperosmotic agent (for example, glycerin, a combination preparation of glycerin and fructose or isosorbide and the like), a ROCK (Rho-kinase) inhibitor (for example, Y-27632 and the like), and an NMDA antagonist.

### EXAMPLES

Hereinafter, the present invention will be described specifically with reference to the Examples, but the present invention is not limited thereto.

2-Propanyl 4-{(3S,5aR,6R,7R,8aS)-6-[(1E,3R)-4-(2,5-difluorophenoxy)-3-hydroxy-1-buten-1-yl]-7-hydroxyoctahydro-2H-cyclopenta[b]oxepin-3-yl}butanoate (Compound A) was used. Compound A can be prepared by a known method. For example, Compound A can be prepared according to Example 1 —> Example 2 —> Example 3 —> Example 4 —> Example 5 —> Example 6 —> Example 7 —> Example 8 —> Example 9 —> Example 10 (1) —> Example 11 —> Example 12 —> Example 13 —> Example 14 —> Example 15 —> Example 16 (25) described in WO 2011/013651.

### Biological Example 1: Intraocular pressure lowering action

Compound A and a β-blocker were used as test substances. Compound A was prepared in a 3 µg/mL solution using a vehicle (an aqueous solution containing 0.3 w/v% sodium citrate, 0.5 w/v% polysorbate 80, and 0.8 w/v% sodium chloride). Timolol (trade name: Timoptol (registered trademark) 0.5% ophthalmic solution, Santen Pharmaceutical Co., Ltd.) was used as a β-blocker.

Approximately one hour before administering the vehicle and a test substance to male dogs (TOYO Beagle, 16 to 42 months old at start of use) that have been sufficiently acclimated in advance, intraocular pressure in both eyes was measured, and the intraocular pressure was set as the intraocular pressure value before instillation. Thereafter, a test substance or the vehicle was administered by instillation into the treated eyes in an amount of 30 µL each, and intraocular pressure in both eyes was measured about 2, 4, 6, 8, and 24 hours after the administration. The intraocular pressure was measured by a pneumatic applanation tonometer (Model 30 Classic, Reichert, Inc.) under the local anesthesia after instilling 0.4% oxybuprocaine hydrochloride (Benoxil (registered trademark) ophthalmic solution 0.4%, Santen Pharmaceutical Co., Ltd.), which is a local anesthetic. The measurement was performed until a stable value was obtained three times successively (a difference between continuously obtained measured values was within 0.5 mmHg). The average of the three measured values obtained was defined as the intraocular pressure value (mmHg) and was displayed to one digit after the decimal point.

The value obtained by subtracting the intraocular pressure value at each measurement time from the intraocular pressure value before test substance and vehicle administration was defined as the intraocular pressure lowering width, and the average value of the intraocular pressure lowering width of each individual at each measurement time was defined as the average intraocular pressure lowering width. The maximum value of the average intraocular pressure lowering width at each measurement time was defined as the maximum intraocular pressure lowering width. In addition, the average intraocular pressure lowering width 24 hours after administration was defined as the intraocular pressure lowering width 24 hours after administration. The results are shown in Table 1. Meanwhile, in the case where there are two drug names in a group name, it means the order of administration. For example, the Compound A - timolol group means that Compound A was administered first, and timolol was administered thereafter into the same eye.

**[Table 1]**

| Group name | Number of cases | Maximum intraocular pressure lowering width (mmHg ± S.E.) | Intraocular pressure lowering width 24 hours after administration (mmHg ± S.E.) | Time after administration exhibiting maximum intraocular pressure lowering action (hours) |
|---|---|---|---|---|
| Vehicle | 8 | 0.7 ± 0.48 | 0.4 ± 0.58 | 6 |
| Vehicle - timolol | 8 | 2.7 ± 0.56 | 0.0 ± 0.56 | 2 |
| Compound A - vehicle | 8 | 5.8 ± 0.35 | 3.5 ± 0.75 | 6 |
| Compound A - timolol | 8 | 7.0 ± 0.52 | 4.8 ± 0.34 | 8 |
| Timolol - Compound A | 8 | 7.1 ± 0.58 | 4.7 ± 0.57 | 8 |

As a result, it was found that the combined use of Compound A and timolol showed a stronger intraocular pressure lowering action compared to each single agent, regardless of the order of administration. Further, it was found that, in the combined use of Compound A and timolol, an intraocular pressure lowering action lasting up to 24 hours after administration was seen, and that there is an effect that the time during which the maximum intraocular pressure lowering action was exerted was longer than that of each single agent.

### INDUSTRIAL APPLICABILITY

The combination of the present invention exerts a significant intraocular pressure lowering action, and is therefore useful for treating glaucoma or ocular hypertension.

## Claims

1. An agent for use in a method of treating glaucoma or ocular hypertension,
the method comprising administering the agent comprising 2-propanyl 4-{(3S,5aR,6R,7R,8aS)-6-[(1E,3R)-4-(2,5-difluorophenoxy)-3-hydroxy-1-buten-1-yl]-7-hydroxyoctahydro-2H-cyclopenta[b]oxepin-3-yl}butanoate as an active ingredient administered in combination with timolol or a pharmaceutically acceptable salt thereof, wherein
timolol or a pharmaceutically acceptable salt thereof and 2-propanyl 4-{(3S,5aR,6R,7R,8aS)-6-[(1E,3R)-4-(2,5-difluorophenoxy)-3-hydroxy-1-buten-1-yl]-7-hydroxyoctahydro-2H-cyclopenta[b]oxepin-3-yl}butanoate are administered separately or concurrently.

2. A pharmaceutical composition for use in a method of treating glaucoma or ocular hypertension, the method comprising administering the pharmaceutical composition comprising 2-propanyl 4-{(3S,5aR,6R,7R,8aS)-6-[(1E,3R)-4-(2,5-difluorophenoxy)-3-hydroxy-1-buten-1-yl]-7-hydroxyoctahydro-2H-cyclopenta[b]oxepin-3-yl}butanoate in combination with timolol or a pharmaceutically acceptable salt thereof.

3. The pharmaceutical composition for use according to claim 2, which is an eye drop or an eye ointment.

4. The pharmaceutical composition for use according to any one of claims 2 or 3, which is a compounding agent.

5. The agent for use according to claim 1 or the pharmaceutical composition for use according to any one of claims 2 to 4, wherein glaucoma is selected from the group consisting of acute angle-closure glaucoma, chronic angle-closure glaucoma, secondary angle-closure glaucoma, primary open-angle glaucoma, secondary open-angle glaucoma, congenital glaucoma, normal tension glaucoma, and hypersecretion glaucoma.

6. The agent for use according to claim 1 or the pharmaceutical composition for use according to any one of claims 2 to 4, wherein the pharmaceutically acceptable salt of timolol is a maleate thereof.

## Patentansprüche

1. Mittel zur Verwendung in einem Verfahren zur Behandlung von Glaukom oder okularem Bluthochdruck,
wobei das Verfahren die Verabreichung des Mittels umfasst, das 2-Propanyl 4-{(3S,5aR,6R,7R,8aS)-6-[(1E,3R)-4-(2,5-difluorophenoxy)-3-hydroxy-1-buten-1-yl]-7-hydroxyoctahydro-2H-cyclopenta[b]oxepin-3-yl}butanoat als Wirkstoff umfasst, der in Kombination mit Timolol oder einem pharmazeutisch akzeptablen Salz davon verabreicht wird, wobei
Timolol oder ein pharmazeutisch akzeptables Salz davon und 2-Propanyl 4-{(3S,5aR,6R,7R,8aS)-6-[(1E,3R)-4-(2,5-difluorophenoxy)-3-hydroxy-1-buten-1-yl]-7-hydroxyoctahydro-2H-cyclopenta[b]oxepin-3-yl}butanoat getrennt oder gleichzeitig verabreicht werden.

2. Pharmazeutische Zusammensetzung zur Verwendung in einem Verfahren zur Behandlung von Glaukom oder okularem Bluthochdruck, wobei das Verfahren die Verabreichung der pharmazeutischen Zusammensetzung umfasst, die 2-Propanyl 4-{(3S,5aR,6R,7R,8aS)-6-[(1E,3R)-4-(2,5-difluorophenoxy)-3-hydroxy-1-buten-1-yl]-7-hydroxyoctahydro-2H-cyclopenta[b]oxepin-3-yl}butanoat in Kombination mit Timolol oder einem pharmazeutisch akzeptablen Salz davon umfasst.

3. Die pharmazeutische Zusammensetzung zur Verwendung gemäß Anspruch 2, die in Form von Augentropfen oder einer Augensalbe ist.

4. Die pharmazeutische Zusammensetzung zur Verwendung gemäß einem der Ansprüche 2 oder 3, die ein Compoundiermittel ist.

5. Das Mittel zur Verwendung gemäß Anspruch 1 oder die pharmazeutische Zusammensetzung zur Verwendung gemäß einem der Ansprüche 2 bis 4, wobei das Glaukom ausgewählt ist aus der Gruppe bestehend aus akutem Winkelschließungsglaukom, chronischem Winkelschließungsglaukom, sekundärem Winkelschließungsglaukom, primärem Offenwinkelglaukom, sekundärem Offenwinkelglaukom, kongenitalem Glaukom, Normalspannungsglaukom und Hypersekretionsglaukom.

6. Das Mittel zur Verwendung gemäß Anspruch 1 oder die pharmazeutische Zusammensetzung zur Verwendung gemäß einem der Ansprüche 2 bis 4, wobei das pharmazeutisch akzeptable Salz von Timolol ein Maleat davon ist.

## Revendications

1. Agent pour l'utilisation dans un procédé de traitement du glaucome ou de l'hypertension oculaire, le procédé comprenant l'administration de l'agent comprenant du 4-{ (3S, 5aR, 6R, 7R, 8aS) -6- [(lE, 3R) -4- (2,5-difluorophénoxy)-3-hydroxy-1-butèn-1-yl]-7-hydroxyoctahydro-2H-cyclopenta[b]oxépin-3-yl}butanoate de 2-propanyle comme ingrédient actif administré en combinaison avec du timolol ou son sel pharmaceutiquement acceptable,
le timolol ou son sel pharmaceutiquement acceptable et le 4-{(3S,5aR,6R,7R,8aS)-6-[(1E,3R)-4-(2,5-difluorophénoxy)-3-hydroxy-1-butèn-1-yl]-7-hydroxyoctahydro-2H-cyclopenta[b]oxépin-3-yl}butanoate de 2-propanyle étant administrés séparément ou simultanément.

2. Composition pharmaceutique pour l'utilisation dans un procédé de traitement du glaucome ou de l'hypertension oculaire, le procédé consistant à administrer la composition pharmaceutique comprenant du 4-{ (3S, 5aR, 6R, 7R, 8aS) -6- [(lE, 3R) -4- (2,5-difluorophénoxy)-3-hydroxy-1-butèn-1-yl]-7-hydroxyoctahydro-2H-cyclopenta[b]oxépin-3-yl}butanoate de 2-propanyle en combinaison avec du timolol ou son sel pharmaceutiquement acceptable.

3. Composition pharmaceutique pour l'utilisation selon la revendication 2, qui est un collyre ou un onguent ophtalmique.

4. Composition pharmaceutique pour l'utilisation selon l'une quelconque des revendications 2 ou 3, qui est un agent de malaxage.

5. Agent pour l'utilisation selon la revendication 1 ou composition pharmaceutique pour l'utilisation selon l'une quelconque des revendications 2 à 4, le glaucome étant sélectionné dans le groupe constitué du glaucome aigu à angle fermé, glaucome chronique à angle fermé, glaucome secondaire à angle fermé, glaucome primaire à angle ouvert, glaucome secondaire à angle ouvert, glaucome congénital, glaucome à pression normale, et glaucome à hypersécrétion.

6. Agent pour l'utilisation selon la revendication 1 ou composition pharmaceutique pour l'utilisation selon l'une quelconque des revendications 2 à 4, le sel pharmaceutiquement acceptable de timolol étant un maléate de celui-ci.
